# EUROPEAN PATENT SPECIFICATION

(11) **EP 2 593 148 B1**
(45) Date of publication and mention of the grant of the patent: **13.04.2016**
(21) Application number: 11741000.1
(22) Date of filing: 15.07.2011
(51) Int. Cl.: A61L 9/12, A61L 9/03

(54) **REFILL FOR AND A METHOD OF INSERTING A REFILL INTO A VOLATILE MATERIAL DISPENSER**
NACHFÜLLEINHEIT UND VERFAHREN ZUR BEFESTIGUNG EINER NACHFÜLLEINHEIT IN EINEM SPENDER FÜR FLÜCHTIGES MATERIAL
UNITÉ DE RECHARGE ET PROCÉDÉ DE FIXATION DES UNITÉS DE RECHARGE SUR UN DISTRIBUTEUR DE MATIÈRE VOLATILE

(30) Priority: 16.07.2010 US 837660
(43) Date of publication of application: 22.05.2013
(73) Proprietor: S.C. Johnson & Son, Inc., Racine, WI 53403 (US)
(72) Inventor: KUBICEK, Chris, A., East Troy WI 53120 (US); GASPER, Thomas, P., Germantown WI 53022 (US)
(74) Representative: Ruschke, Hans Edvard
(86) International application number: PCT/US2011/001254
(87) International publication number: WO 2012/009018

(56) References cited:
- EP-A1- 1 175 833
- WO-A1-2004/082727
- GB-A- 2 411 590
- US-A- 5 222 186
- US-A1- 2002 172 512
- US-A1- 2003 189 022
- US-A1- 2004 247 301

## Description

### 1. Field of the Invention

The present invention generally relates to a refill containing a volatile material. More particularly, the present invention relates to a refill adapted to be functionally coupled with a volatile material dispenser.

### 2. Description of the Background of the Invention

Multiple different volatile material dispensers are commercially sold and generally include a housing and a volatile material refill that is inserted into the housing. The refill generally, includes a container or bottle for holding a volatile material therein. In some dispensers, the volatile material is passively emitted therefrom. In other dispensers, a diffusion element is utilized to facilitate the dispensing of the volatile material. Examples of diffusion elements include heaters, piezoelectric elements, fans, aerosol actuators, and the like. Regardless of the manner in which the volatile material is emitted, once the volatile material has been expended from the refill, the refill can typically be removed by a user and replaced with a new refill.

One type of commercial volatile material dispenser, referred to herein as a plug-in scented oil dispenser, includes a housing and a heater disposed within the housing. A refill for use with a plug-in scented oil dispenser generally includes a container portion having a bottom end and a top end, wherein the container portion terminates in a neck portion at the top end. A volatile material is disposed within the container portion and a wick is in contact with the volatile material and extends out of the refill through the neck portion. A plug or other connector generally positions and retains the wick within the neck portion. A cap covers the wick and the neck portion and may be removed before the container is inserted into the dispenser. Upon insertion of the refill into the dispenser, at least a portion of the wick is disposed adjacent the heater such that volatile material that moves through the wick is volatilized by the heater.

Another feature of various volatile material dispensers is a retention mechanism. Generally, a refill will have a shape and size complementary to the dimensions of the dispenser housing, and many dispenser housings have mechanisms designed to securely retain a refill container within the housing. Some of the retention mechanisms may only work properly with specific refill containers that exhibit certain structural features.

US2002/172512 discloses a liquid vaporizer which includes a housing stabilization system for stabilizing the vaporizer when it is plugged into a wall outlet, wherein the stabilization system comprises one or more projections which abut the wall or outlet to which the vaporizer is inserted, and wherein the liquid vaporizer stabilization system may also serve to prevent dispensing of vaporized material proximate to the electrical outlet.

US 2003/189022 discloses a universal bottle that interfits into housings of different commercial liquid vaporization devices and which has a neck having an opening and two sets of opposite external surfaces that are dimensioned so that in one orientation, the bottle will fit into one of the commercial devices, and, by rotating the bottle a predetermined amount to another orientation, the bottle can be operatively inserted into the other of the commercial devices, wherein, in each instance, a locking system on the bottle cooperates with the housing of the commercial device to retain the bottle in its operative position.

EP 1 175 833 discloses an electric evaporator for insecticides or perfumes in liquid formulation contained in a bottle which can be steadily inserted into a container, comprising a wick, an element for housing and centring said wick and a respective electric heating device, wherein the wick housing and centring element are joined to the walls of the container through elastic bridge-pieces which have the shape of a loop extending respectively in horizontal and vertical planes, wherein a cam device which can be actuated manually outside of the container allows displacement of the wick housing and centring element in a direction perpendicular to the axis of the wick, towards or away from said heating device, and wherein the evaporator comprises a system for snapfastening and locking the bottle with regard to tampering by children.

US 5,222,186 discloses an electrical apparatus for vaporizing active substances, perfumes or the like volatile substances, consisting of a housing having an electrical heating means and a container for liquid to be vaporized connectable to the housing, in the container a wick or the like being mounted by means of which the liquid is supplied to the heating means, wherein the wick passes through the heating means at a through passage adapted to the wick, the heating means further consisting of a ceramic heating body with electrical heating coil let into said body, wherein the heating body comprises a recess which extends tangentially to its through passage and which is made rectilinear and into which the heating coil is inserted, and which is filled with potting composition, the electrical leads of the heating coil being led out of the heating body substantially coaxially to the heating coil.

GB 2411590 discloses an emanator device having a bottle and a wick for transferring fluid to a dispersing section. Engagement means including a thread incorporating gaps is provided between the bottle and the dispensing section.

### BRIEF SUMMARY OF THE INVENTION

According to a first aspect of the invention there is provided a refill for a volatile material dispenser as defined in claim 1. Optional features of this aspect are defined in dependent claims 2 and 3. The described arrangement of the refill for a volatile material dispenser comprises a container having a neck portion. Also described is a wick in contact with a volatile material in the container which extends out of the neck portion. A structure is disposed on an outer surface of the neck portion for attachment of a cap thereto. The structure is also adapted to interact with latches disposed in a volatile material dispenser to retain the refill therein, and the container is devoid of an additional retaining structure on the neck portion thereof.

A second aspect the invention provides a method as defined in claim 4. Optional features of this aspect are defined in dependent claims 5 to 7. Thus described is a method of inserting a refill into a volatile material dispenser including the step of providing a volatile material dispenser. Also described is a method further including the step of providing a refill having a container, a neck portion, a wick in contact with volatile material in the container and extending out the neck portion, an annular member disposed on an outer surface of the neck portion, and a cap attached to the neck portion by the annular member. Additionally, the method includes the step of removing the cap and inserting the refill into the dispenser such that at least one latch in the dispenser grasps the annular member on the neck portion of the container and retains the container therein.

Also described is a method of inserting a refill into a volatile material dispenser including the step of providing a volatile material dispenser with at least one latch. The method further includes the step of providing a refill having a container with volatile material, a wick in contact with the volatile material and extending out a neck portion of the container, and a cap disposed over the wick and connected to an annular member disposed on the neck portion. Additionally, the method includes the steps of disconnecting the cap from the refill and inserting the refill into a cavity formed within the volatile material dispenser such that the at least one latch engages the annular member to retain the container therein. The neck portion of the refill is devoid of any other structure that functions to retain the refill.

### BRIEF DESCRIPTION OF THE DRAWINGS

FIG. 1 illustrates a top isometric view of a refill according to one embodiment of the present invention;
FIG. 2 is a front elevational view of the refill of FIG. 1;
FIG. 3 is a rear elevational view of the refill of FIG. 1;
FIG. 4 is a side elevational view of the refill of FIG. 1;
FIG. 5 is an exploded view of the refil of FIG. 1 including a cap;
FIG. 6 is a cross-sectional view of the refill of FIG. 1 taken generally along the lines 6-6 of FIG. 4, with the cap disposed on the refill;
FIG. 7 is a top isometric view of a refill according to a further embodiment of the present invention;
FIG. 8 is a front elevational view of the refill of FIG. 7;
FIG. 9 is a top isometric view of a refill not according to the present invention;
FIG. 10 is a top isometric view of a volatile material dispenser; and
FIG. 11 is a cross-sectional view of the volatile material dispenser of FIG. 10 taken generally along the lines 11-11 of FIG. 10 with an uncapped version of the refill of FIG. 6 inserted therein.

Other aspects and advantages of the present invention will become apparent upon consideration of the following detailed description.

### DETAILED DESCRIPTION OF THE INVENTION

The present invention is directed to refills for holding volatile materials. While the present invention may be embodied in many different forms, several specific embodiments are discussed herein with the understanding that the present invention is to be considered only as an exemplification of the principles of the invention, and it is not intended to limit the invention to the embodiments illustrated.

Further, the use of the term volatile material herein refers to any volatile material that a consumer may desire to emit into an area surrounding one or more refills holding the volatile material(s) and/or a dispenser holding one or more refills. Illustratively, the types of volatile materials may be, for example, a cleaner, an insecticide, an insect repellant, an insect attractant, a mold or mildew inhibitor, a fragrance, a disinfectant, an air purifier, an aromatherapy scent, an antiseptic, a positive fragrancing volatile material, an air-freshener, a deodorizer, or the like, and combinations thereof. Additives may be included in the volatile material, such as, for example, fragrances, and/or preservatives.

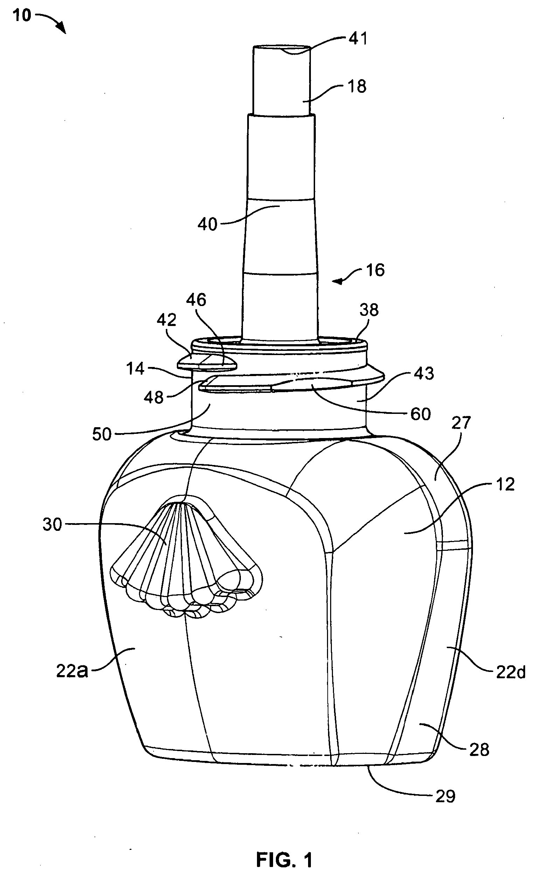

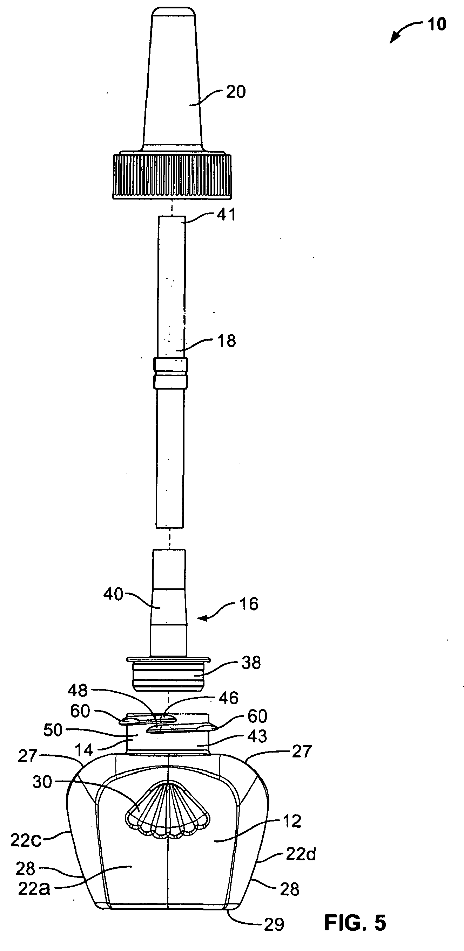

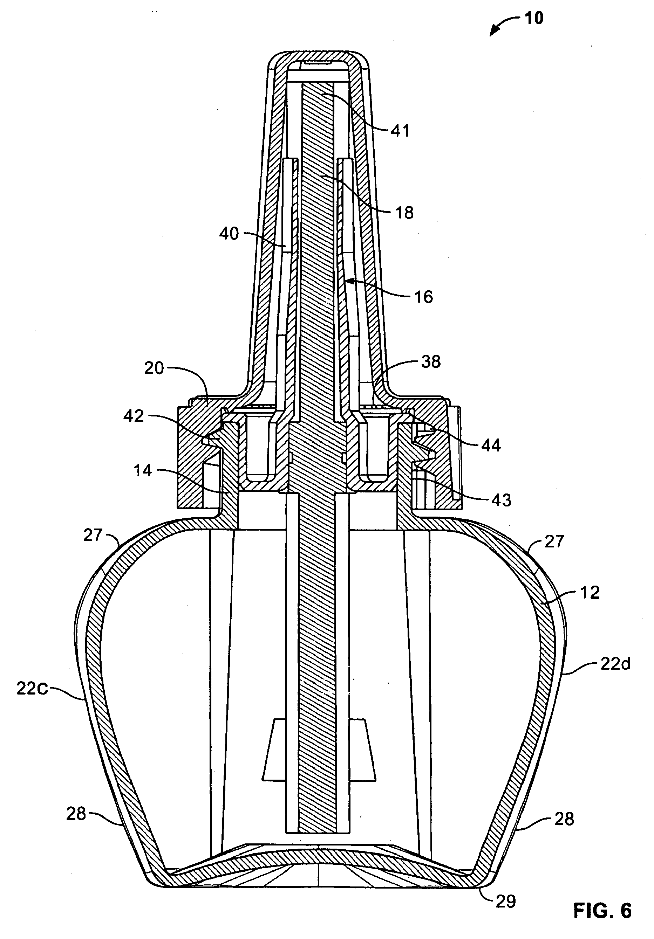

FIGS. 1-5 illustrate a refill 10 according to one embodiment of the present invention. The refill 10 generally includes a container portion 12 that holds a volatile material, wherein a generally cylindrical neck portion 14 extends upwardly from the container portion 12. A plug assembly 16 is disposed within and attached to the neck portion 14 of the refill 10. A wick 18 is disposed in contact with the volatile material inside the container portion 12 and extends upwardly through the neck portion 14, such that a portion of the wick 18 may be exposed to a surrounding environment. The wick 18 is retained within the neck portion 14 by the plug assembly 16. A cap 20, as seen in FIGS. 5 and 6, may be threaded onto the neck portion 14 and disposed over the wick 18 and plug assembly 16 during shipment and storage thereof, as discussed in greater detail hereinafter.

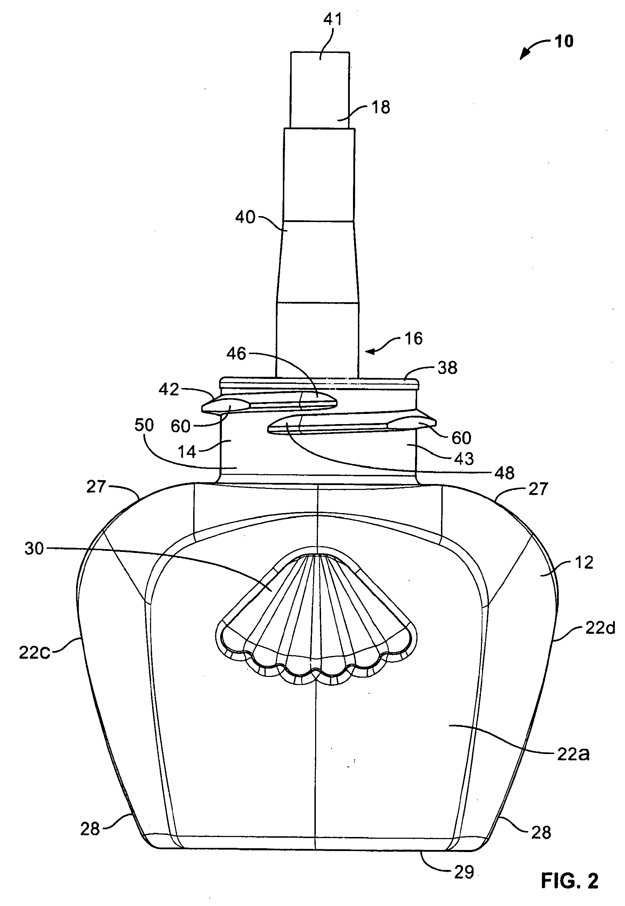

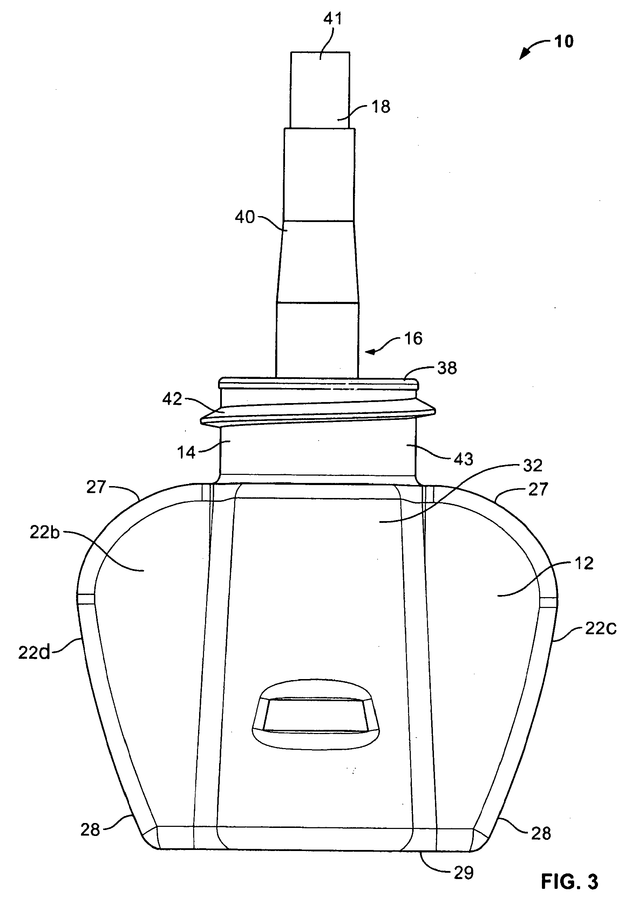

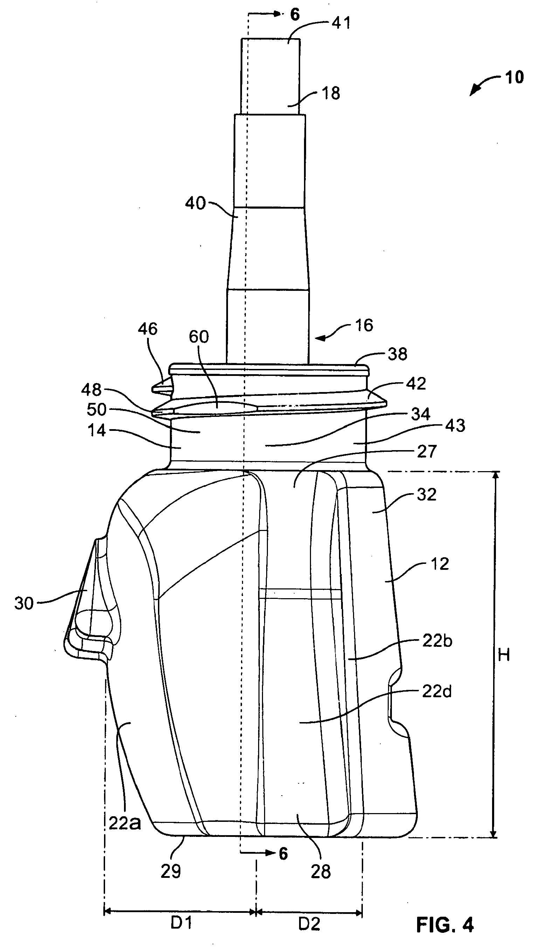

The container portion 12 includes front and rear surfaces 22a, 22b and first and second side surfaces 22c, 22d connecting the front and rear surfaces 22a, 22b. Referring to FIGS. 1 and 4, the front surface 22a has a generally bulbous central portion and is generally curved inwardly at sides and a bottom thereof and the rear surface 22b is generally planar. Further, as seen in FIGS. 2 and 3, the side surfaces 22c, 22d begin at the neck portion 14 and curve outwardly at top portions 27 thereof and inwardly at bottom portions 28 thereof to generally form a heart shape that is truncated at a bottom surface 29 thereof.

A shell-shaped protrusion 30 extends outwardly from the front surface 22a and a semi-cylindrical projection 32 extends outwardly from the rear surface 22b along a height H (FIG. 4) of the container portion 12. Referring to FIG. 4, the front and rear surface 22a, 22b are offset with respect to a centerpoint 34 of the neck portion 14. In particular, the front surface 22a is spaced a distance D1 from the centerpoint 34 and the rear surface 22b is spaced a distance D2 from the centerpoint 34, wherein D1 is greater than D2.

Although the container portion 12 is depicted herein as having a particular size, shape, and configuration, the size, shape, and/or configuration of the container portion 12 may be varied without departing from the scope of the present invention.

As noted above, the plug assembly 16 is attached to the neck portion 14 of the refill 10 and surrounds and retains the wick 18. As best seen in FIGS. 5 and 6, the plug assembly 16 includes a retention portion 38 that snaps onto the neck portion 14 of the refill 10 and a sheath portion 40 that extends upwardly from the retention portion 38 and surrounds the wick 18. The sheath portion 40 extends around a majority of a portion of the wick 18 that extends out of the refill 10, leaving a top portion 41 of the wick 18 exposed.

As best seen in FIGS. 1, 2, and 6, a thread 42 extends outwardly from an outer surface 43 of the neck portion 14 of the refill 10 to interact with and retain the cap 20. The thread 42 spirals down around the neck portion 14 of the refill 10 and engages a similarly shaped threaded groove 44 (FIG. 6) in the cap 20 to retain the cap 20 thereon. The thread 42 includes an upper end 46 that spirals down around the neck portion 14 of the refill 10 and terminates at a lower end 48 of the thread 42. Both upper and lower thread ends 46, 48 are disposed on a front portion 50 of the neck portion 14. Optionally, the upper and lower thread ends 46, 48 may be disposed at a different position on the neck portion 14 and/or may be disposed at positions different from one another. As can be seen from FIGS. 1-3, the thread 42 extends a little more than 360 degrees around the neck portion 14 of the refill 10. The thread 42 preferably extends between about 180 degrees and about 1440 degrees about the neck portion 14, more preferably extends between about 270 degrees and about 450 degrees about the neck portion 14, and most preferably extends about 360 degrees about the neck portion 14. A thread 42 extension of at least 180 degrees is necessary to insure proper placement of the top portion 41 of the wick 18 with respect to a heater disposed in a dispenser, but an extension of 360 is preferable to insure proper placement of the top portion 41 of the wick 18 and consistent refill 10 height placement. If multiple threads are disposed on the neck portion 14, multiple attachment areas are provided. Referring to FIGS. 2 and 5, the thread 42 includes a number of voids 60 therein, wherein the voids allow the refill 10 to interact with attachment mechanisms in one or more dispensers without interference therewith. Although two voids 60 are depicted, any number of voids 60 may be utilized. As is evident from FIGS. 1-6, the neck portion 14 of the refill 10 is devoid of any other projections, annular members, or other features thereon that would allow the refill 10 to be retained within a volatile material dispenser.

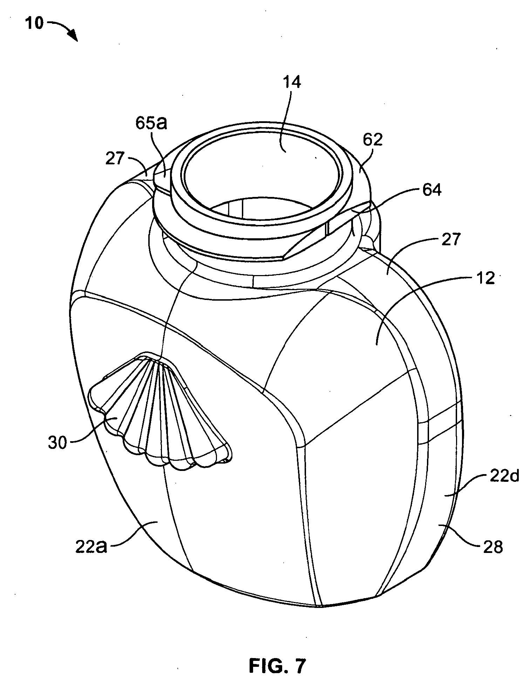

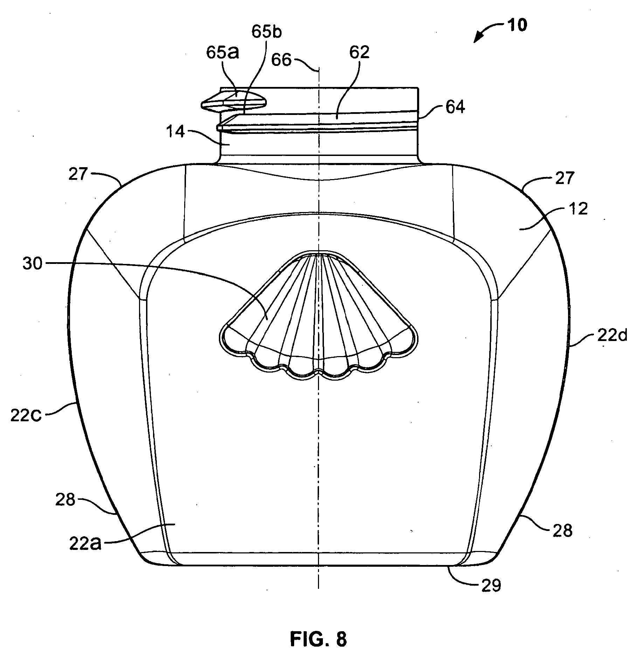

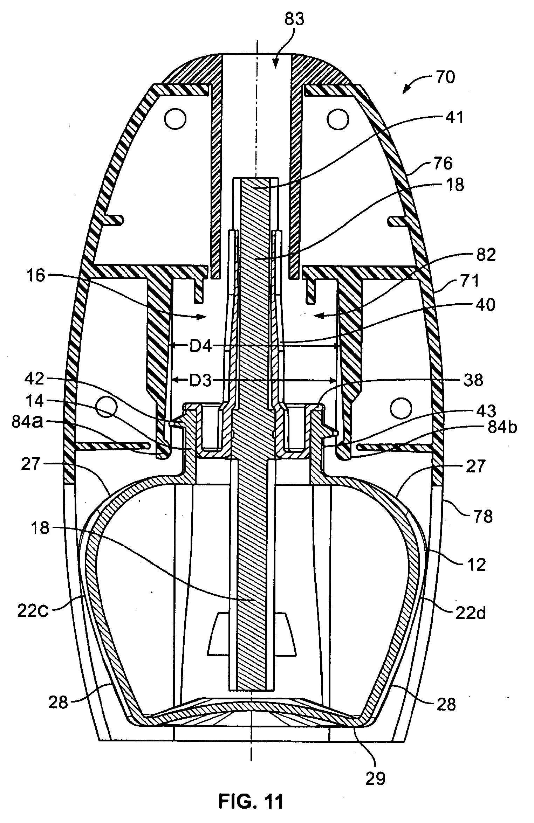

Although the thread 42 is shown as continuous, the thread 42 may alternatively be discontinuous, as depicted in FIGS. 7 and 8. In particular, the refill 10 of FIGS. 7 and 8 is identical to the refill 10 of FIGS. 1-6, except for a thread 62 thereof. The thread 62 includes a gap or void 64 therein and also starts and begins at positions different from the thread 42 (the thread 62 start and end points 65a, 65b are offset to the left of a central axis 66 of the refill 10). The thread 62 may extend about the neck portion 14 of the refill 10 of FIGS. 7 and 8 the same distances as detailed with respect to the thread 42. The gap 64 in the thread 62 provides a manner to more evenly hold the refill 10 in place. Specifically, as can be seen, the thread 62 is spiral in nature and one side of the thread 62 is higher than an opposing side of the thread 62. If, for example, opposing latches (see latches in relation to FIG. 11) are utilized, a first of the latches would abut the neck portion 14 at the gap 64 to provide a friction fit therebetween and a second of the latches would hook under and retain the thread 62 at a point opposite the gap 64. The gap 64 allows abutment of the latch at a point directly opposite the thread 62 (at the same vertical position), rather than above or below such latch 62. Otherwise, it is possible in some situations, that opposing latches would grasp and retain portions of the thread 62 opposite one another that are at different vertical positions, thereby causing the refill 10 to be tilted.

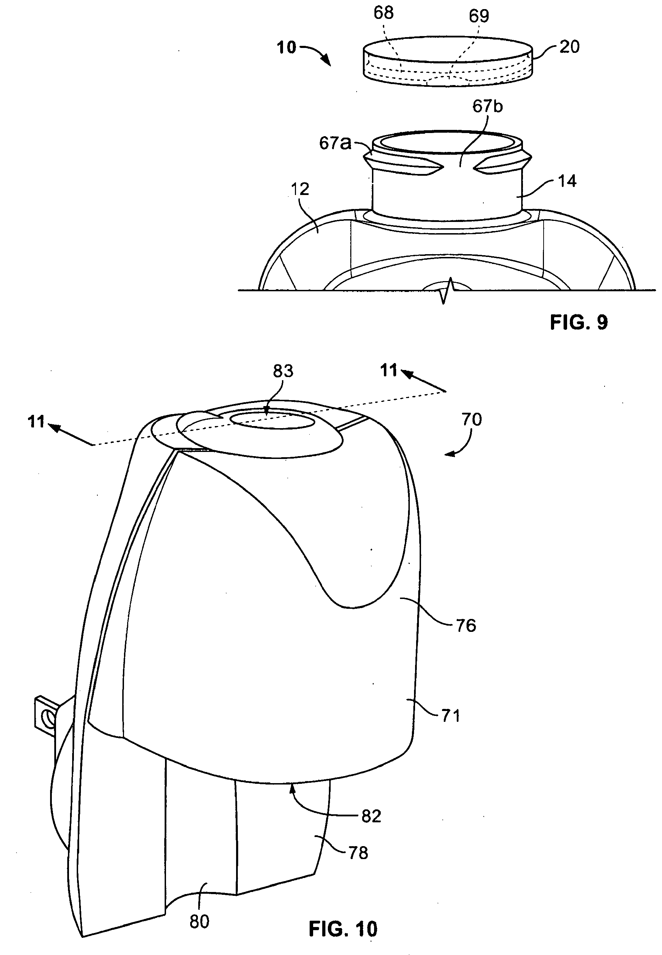

In a further aspect as seen in FIG. 9, a non-spiraled, annular snap 67a is disposed on the neck portion 14 of the refill 10 with a small gap 67b between ends thereof and a groove 68 is disposed around an entire inner surface of the cap 20. The cap 20 optionally includes a projection 69 extending from the inner surface thereof, wherein the projection 69 must be aligned with the gap 67b in the annular snap to remove the cap 20 from the refill 10. As with previous embodiments, the annular snap 67a would also serve to retain the refill 10 within a dispenser. Other types of annular snaps are possible, wherein the annular snap extends between about 180 degrees and about 360 degrees about the neck portion 14 of the refill 10. For example, in a further aspect simitar to that of FIG. 9, the annular snap 67a extends 360 degrees around the neck portion 14 and the projection 69 is removed. Still optionally, one or more continuous or discontinuous projections that snap fit with the annular snap may be disposed on an inner surface of the cap. Again, the annular snap also retains the refill 10 within a dispenser.

FIGS. 10 and 11 illustrate one volatile material dispenser 70 that accepts and retains the refill 10. The dispenser 70 includes a housing 71 having an upper portion 76 and a lower portion 78. A semi-cylindrical shaped groove 80 is disposed in the lower portion 78 of the dispenser 70 and a cavity 82 is formed within the upper portion 76 of the dispenser 70, wherein a refill, such as the refill 10, may be inserted into and retained within the cavity 82. A channel 83 is disposed within an upper portion of the cavity 82 and a set of opposing resilient latches 84a, 84b is also disposed within a lower portion of the cavity 82. A heater (not shown) is generally disposed adjacent the channel 83 for heating a wick disposed within the channel 83, as discussed in greater detail hereinafter.

Before use of the refill 10, the cap 20 is unthreaded and removed from the neck portion 14 of the refill 10. After the cap 20 is removed, the refill 10 may be inserted into a dispenser, such as the dispenser 70 of FIGS. 10 and 11, by inserting the top portion 41 of the wick 18 into the channel 83 formed within the cavity 82 of the dispenser 70 and moving the refill 10 into the dispenser 70. As the refill 10 is inserted, the semi-cylindrical projection 32 rides within the similarly-shaped groove 80 in the lower portion 78 of the dispenser 70 and the wick 18 moves further into the channel 83. Eventually, the thread 42 interferes with the opposing resilient latches 84a, 84b disposed within the lower portion of the cavity 82. The thread 42 pushes the resilient latches 84a, 84b outwardly until the thread 42 passes the latches 84a, 84b. Once the thread 42 passes the latches 84a, 84b, the latches 84a, 84b return to their original position, such that when gravity acts on the refill 10, the thread 42 interferes with the opposing resilient latches 84a, 84b to prevent downward movement of the refill 10. In particular, as seen in FIG. 11, when the latches 84a, 84b are in a resting position, a distance D3 between innermost points of the latches 84a, 84b is less than a distance D4 between outermost edges of the thread 42. The refill 10 may be removed from the dispenser 70 by pulling downwardly on the refill 10, which thereby pushes outwardly on the latches 84a, 84b to allow removal of the refill 10. By using the same thread 42 to both retain the cap 20 on the refill 10 and retain the refill 10 within the dispensing device 70, manufacturers may save time, money, and material.

### INDUSTRIAL APPLICABILITY

Many contemporary refills use at least one retention mechanism to retain the cap on the refill and at least one additional and separate retention mechanism to retain the refill within the dispenser. However, the present invention uses just one retention mechanism to retain both the cap on the refill and the refill within the dispensing device. Thus, the present invention saves time, money and material in the manufacturing process by reducing the number of necessary parts.

## Claims

1. A refill (10) for a volatile material dispenser, comprising:
a container (12) having a neck portion (14);
a wick (18) in contact with a volatile material in the container and extending out the neck portion; and
an annular, spiraled thread (42, 62) disposed on an outer surface of the neck portion (14) of the container (12) for attachment of a cap (20) thereto;
**characterized in that** the thread (42, 62) is also adapted to interact with latches (84a, 84b) disposed in a volatile material dispenser (70) to retain the refill therein, wherein the container is devoid of an additional retaining structure on the neck portion thereof;
further **characterized in that** the thread (42, 62) includes at least one void (60, 64) therein.

2. The refill of claim 1, wherein the thread (42, 62) extends between about 270 degrees and about 450 degrees around the neck portion (14).

3. The refill of claim 2, wherein the thread (42, 62) extends about 360 degrees around the neck portion (14).

4. A method of inserting a refill (10) into a volatile material dispenser (70), the method comprising the steps of:
providing a volatile material dispenser (70);
providing a refill (10) having a container (12), a neck portion (14), a wick (18) in contact with volatile material in the container and extending out the neck portion (14), a thread (42, 62) disposed on an outer surface of the neck portion, the thread including at least one void (60, 64) therein, and a cap (20) attached to the neck portion (14) by the thread (42, 62); and
removing the cap and inserting the refill into the dispenser such that two opposing latches (84a, 84b) in the dispenser grasp the thread on the neck portion (14) of the container (12) and retain the container therein.

5. The method of claim 4, wherein the container (12) is retained by an interference between lower portions of the thread (42, 62) and an upper portion of the latch (84a, 84b).

6. The method of claim 4, wherein the step of providing the refill (10) further includes the step of providing (42, 62) the thread on the neck portion (14) to extend between about 270 degrees and about 450 degrees around the neck portion.

7. The method of claim 6, wherein the thread (42, 62) extends about 360 degrees around the neck portion (14).

## Patentansprüche

1. Nachfülleinheit (10) für einen Spender für flüchtige Substanz, mit:
einem Behälter (12) mit einem Halsbereich (14);
einem Docht (18), der in Kontakt mit einem flüchtigen Stoff im Behälter steht und aus dem Halsbereich ragt; und
einem wendelartig umlaufenden Gewindegang (42, 62) auf einer Außenfläche des Halsbereichs (14) des Behälters (12) zum Ansetzen einer Kappe (20);
**dadurch gekennzeichnet, dass** der Gewindegang (42, 62) ausgeführt ist zur Wechselwirkung mit in einem Spender (70) für flüchtigen Stoff angeordneten Klinken (84a, 84b), um den Nachfüllbehälter darin zu arretieren, wobei der Behälter auf seinem Halsbereich keine zusätzlichen Rückhalteelemente aufweist; und
weiterhin **dadurch gekennzeichnet, dass** der Gewindegang (42, 62) mindestens eine Ausnehmung bzw. Leerstelle (60, 64) enthält.

2. Nachfülleinheit nach Anspruch 1, bei der der Gewindegang (42, 62) zwischen etwa 270 Grad und etwa 50 Grad um den Halsbereich (14) verläuft.

3. Nachfülleinheit nach Anspruch 2, bei der der Gewindegang (42, 62) mit etwa 360 Grad um den Halsbereich (14) verläuft.

4. Verfahren zum Einsetzen einer Nachfülleinheit (10) in einen Spender (70) für flüchtige Stoffe, mit folgenden Schritten:
Bereitstellen eines Spenders (70) für flüchtige Stoffe;
Bereitstellen einer Nachfülleinheit (10) mit einem Behälter (12), einem Halsbereich (14), einem in Kontakt mit flüchtigem Stoff im Behälter stehenden Docht (18), der aus dem Halsbereich (14) hinaus ragt; einem Gewindegang (42, 62) auf einer Außenseite des Halsbereichs, wobei der Gewindegang mindestens eine Leerstelle (60, 64) enthält, und mit einer Kappe (20), die mittels des Gewindegangs (42, 62) an den Halsbereich (14) angesetzt ist; und
Abnehmen der Kappe und Einsetzen der Nachfülleinheit in den Spender derart, dass zwei gegenüberliegende Klinken (84a, 84b) im Spender den Gewindegang auf dem Halsbereich (14) des Behälters (12) erfassen und den Behälter dort rückhalten.

5. Verfahren nach Anspruch 4, bei dem der Behälter (12) durch gegenseitigen Eingriff zwischen unteren Teilen des Gewindegangs (42, 62) und einem oberen Teil der Klinke (84a, 84b) rückgehalten wird.

6. Verfahren nach Anspruch 4, bei dem das Bereitstellen der Nachfülleinheit (10) weiterhin beinhaltet, den Gewindegang (42, 62) auf dem Halsbereich (14) so bereitzustellen, dass er zwischen etwa 270 Grad und etwa 450 Grad um den Halsbereich verläuft.

7. Verfahren nach Anspruch 6, bei dem der Gewindegang (42, 62) mit etwa 360 Grad um den Halsbereich (14) verläuft.

## Revendications

1. Recharge (10) pour un distributeur de matière volatile, comprenant :
un récipient (12) ayant une partie de col (14) ;
une mèche (18) en contact avec une matière volatile dans le récipient et s'étendant à l'extérieur de la partie de col ; et
un filet annulaire, en spirale (42, 62) disposé sur une surface extérieure de la partie de col (14) du récipient (12) pour la fixation d'un capuchon (20) à celui-ci ;
**caractérisée en ce que** le filet (42, 62) est également adapté pour interagir avec des taquets (84a, 84b) disposés dans un distributeur de matière volatile (70) pour retenir la recharge dans celui-ci, dans lequel le récipient est dépourvu d'une structure de retenue supplémentaire sur la partie de col de celui-ci ;
**caractérisée en outre en ce que** le filet (42, 62) inclut au moins un vide (60, 64) dans celui-ci.

2. Recharge selon la revendication 1, dans laquelle le filet (42, 62) s'étend entre environ 270 degrés et environ 450 degrés autour de la partie de col (14).

3. Recharge selon la revendication 2, dans laquelle le filet (42, 62) s'étend environ 360 degrés autour de la partie de col (14).

4. Procédé d'insertion d'une recharge (10) dans un distributeur de matière volatile (70), le procédé comprenant les étapes de :
fournir un distributeur de matière volatile (70) ;
fournir une recharge (10) ayant un récipient (12), une partie de col (14), une mèche (18) en contact avec de la matière volatile dans le récipient et s'étendant à l'extérieur de la partie de col (14), un filet (42, 62) disposé sur une surface extérieure de la partie de col, le filet incluant au moins un vide (60, 64) dans celui-ci, et un capuchon (20) fixé à la partie de col (14) par le filet (42, 62) ; et
retirer le capuchon et insérer la recharge dans le distributeur de telle sorte que deux taquets opposés (84a, 84b) dans le distributeur saisissent le filet sur la partie de col (14) du récipient (12) et retiennent le récipient dans ceux-ci.

5. Procédé selon la revendication 4, dans lequel le récipient (12) est retenu par une interférence entre des parties inférieures du filet (42, 62) et une partie supérieure du taquet (84a, 84b).

6. Procédé selon la revendication 4, dans lequel l'étape de fourniture de la recharge (10) inclut en outre l'étape de fourniture (42, 62) du filet sur la partie de col (14) pour s'étendre entre environ 270 degrés et environ 450 degrés autour de la partie de col.

7. Procédé selon la revendication 6, dans lequel le filet (42, 62) s'étend environ 360 degrés autour de la partie de col (14).
